# EUROPEAN PATENT SPECIFICATION

(11) **EP 3 396 495 B1**
(45) Date of publication and mention of the grant of the patent: **08.12.2021**
(21) Application number: 17864078.5
(22) Date of filing: 29.03.2017
(51) Int. Cl.: G06F 3/01

(54) **NEUROCOMPUTER SYSTEM FOR SELECTING COMMANDS ON THE BASIS OF RECORDING BRAIN ACTIVITY**
NEUROCOMPUTERSYSTEM ZUR AUSWAHL VON BEFEHLEN AUF GRUNDLAGE DER AUFZEICHNUNG DER GEHIRNAKTIVITÄT
SYSTÈME INFORMATIQUE NEURONAL POUR CHOISIR DES INSTRUCTIONS SUR LA BASE D'ENREGISTREMENT DE L'ACTIVITÉ CÉRÉBRALE

(30) Priority: 28.10.2016 RU 2016142493
(43) Date of publication of application: 31.10.2018
(73) Proprietor: NEUROCHAT LIMITED LIABILITY COMPANY, 121205, Moscow (RU)
(72) Inventor: KAPLAN, Aleksandr Yakovlevich, Moscow 117630 (RU); LIBURKINA, Sofya Pavlovna, Moscow 119002 (RU); GANIN, Ilya Petrovich, Moscow 111673 (RU); GRIGORYAN, Rafal Karenovich, Moscow 117279 (RU); GALKINA, Nataliya Valentinovna, Moscow 125195 (RU); MUSTAFIN, Yuriy Renatovich, Moscow 107589 (RU); LUZHIN, Aleksandr Olgerdovich, Moscow 123022 (RU)
(74) Representative: Gallego Jiménez, José Fernando
(86) International application number: PCT/RU2017/000183
(87) International publication number: WO 2018/080336

(56) References cited:
- WO-A1-2012/013535
- CN-A- 103 995 582
- US-A1- 2011 152 710
- US-A1- 2012 059 273
- US-A1- 2013 130 799
- US-B1- 9 389 685

## Description

This invention relates to neurocomputer systems, namely to the field of contactless control of electronic computing or other technical devices using brain-computer interfaces to select commands. It can be used in communication and control systems, particularly for people with impaired motor function, as well as healthy people in situations when commands are selected in contactless mode.

There is the method for controlling devices by eye gestures (see RU 2522848 published on July 20, 2014), when at least one control position is specified for the user, at least one eye gesture is associated with each control position, one command is associated with each combination of control position and eye gesture associated with it, conditions are created under which specific visual stimuli appear in control positions at different times, the user is given instruction for shifting gaze to control position associated with required command and making eye gesture after appearance of specific visual stimulus in it, the user's eye gestures are recorded, the user's eye movements are analyzed in order to identify the eye gesture and determine the time of its making, and if the eye gesture is identified and the time of its making complies with the time of stimulus appearance in one control position, the giving of the command associated with the combination of this eye gesture and this control position is detected and it is delivered to the controlled device. The disadvantage of this method is the need to use eye gestures, the making of which can be limited for certain categories of users, including users with impaired motor function.

The patent application document with publication number CN103995582A describes a brain-computer interface character input method and system based on steady-state visual evoked potential (SSVEP). CN103995582A describes that input characters are divided into character sets, wherein characters arranged in the character sets are selected through the same key. The system as described in CN103995582A comprises an LED visual stimulator, an electroencephalography acquisition platform, a PC real-time processing system and a character input interface.

The patent application document with publication number US2013/0130799 describes a computerized method for decoding visual evoked potentials, the method involving obtaining a set of brain activity signals being recorded from a subject's brain during displaying a set of targets on a display, at least one target being modulated periodically at a target-specific modulation parameter and decoding a visual evoked potential (VEP) from the brain activity signals. US2013/0130799 also describes that the brain activity signals may be encephalogram signals captured at the subject's scalp during said displaying using one or more electrodes.

There is a neurocomputer system (see US 9389685 published on July 12, 2016) that consists of user interface, including electrodes configured to detect brain waves, camera that allows taking pictures of surrounding space with the object for which the user is interested in performing the action, computer intended to identify boundaries of objects within the image, divide the image by grid, consisting of rows and columns structured so that the edges of rows and columns, in general, match with boundaries of objects within the image, demonstrate the image with the overlaying grid to the user. The system is equipped with means that allow the user to select a certain cell located at the intersection of row and column in the grid. The user selects the cell by the brain-computer interface (hereinafter IMK), which operates on the basis of P300 technology. The disadvantage of this system is relatively low speed and significant amount of time required for complete search of various cells within the grid, which is especially important for large objects, regarding which the user is interested in performing the action. Besides, if there are a lot of commands in IMK-P300, the probability of unintentional shifting of the selection to non-target commands increases, as well as the role of distracting factors increases, which leads to increase in the number of command selection errors that reduce the efficiency of the entire system.

In general, the functioning of IMK-P300 is based on the analysis of evoked potentials of the human brain, or event-related potentials. Such potentials are specific brain response to rare or significant (for user) stimulus that can be obtained after the procedure of processing the recorded electroencephalogram. In most cases, the visual modality is used for stimuli in communication systems based on IMK-P300 (for example, the appearance of images or symbols on the screen, or their highlighting that is increasing the brightness). The neurocontrol system based on IMK-P300 is organized as follows. Generally, the user sees the screen with symbols, representing various control commands (including letters, if the interface is intended for typing texts), commonly organized as matrix table. Individual elements-symbols of this matrix are alternately highlighted in random order. The user should focus his or her attention on the symbol-command, which he or she wants to select using the interface. Every time this symbol highlights, the user should mentally respond to this event (for example, by calculating the total number of highlights of this symbol). Throughout the process, the electroencephalogram is recorded for the user, and the program compares the brain responses to the highlighting of different symbols. The specific pattern is observed in responses to the target symbol-command highlighting. It is so-called P300 wave, which leads to the increase in signal amplitude for this symbol. The purpose of the algorithm is to recognize such intense response to the highlighting of the symbol required to the user. After that the program determines that this particular symbol-command was in the focus of the user's attention and specifies it as the target one. Thus, it becomes possible to select any command from the whole set of symbols-commands presented on the screen (Farwell L.A., Donchin E. Talking off the top of your head: toward a mental prosthesis utilizing event-related brain potentials. EEG a. Clin. Neurophysiol. 1988. 70: 510-523.). For example, as shown in US 9389685, imaginary grid overlaying the object image or other visual environment modules, providing the use of visual modality for generation of recorded stimulating pulses, can be used as the screen.

Command selection when using IMK-P300 can take up to several tens of seconds. It is primarily determined by the number of elements used to generate the recorded stimulating pulses for certain commands and the number of repetitions used to improve the reliability of identifying the stimulus selection by the user. The relationship of these indicators and the time of command selection was thoroughly studied in literature for visual stimulus environment represented by letter-symbol matrices with different number of contained elements. For example, the work by Guger et al. (2009) on large sample of test subjects (N = 100) included studying the possibility of control in IMK-P300 with matrix size of 6x6. The time of one command selection was about 29 seconds with average command accuracy of 91% (73% of test subjects reached 100% accuracy). This time was observed at 15 repetitions of highlighting cycles in IMK-P300. The time of selection can be reduced by factor of 1.5-3 with decrease in the number of repetitions, but this usually leads to degradation of accuracy, since the probability of error is lower at greater number of stimuli repetitions. The work speed at command selection in 29 seconds is quite slow and tedious for the user, especially taking into account that the matrix size of 6x6 (36 commands), as a rule, is not enough for full communication.

Thus, the use of IMK-P300 is characterized by low command selection speed and high user fatigue for visual stimulus environments, containing significant number of elements used to generate recorded stimulating pulses for specific commands.

The major goal of this invention is to overcome the above-mentioned disadvantages and improve the ergonomics of the system as a whole

The scope of the invention is defined by the appended claims.

A technical result achieved by the proposed invention consists in reducing the time needed to select the element in visual stimulus environment, containing large number of elements, while maintaining specific level of selection reliability.

The specified technical result is achieved due to the fact that the neurocomputer system for selecting commands based on brain activity recording includes
- an electroencephalograph;
- electrodes configured to transmit detected pulses;
- an electronic computing device for analyzing the recorded brain activity; and
- a visual stimulus module that includes a screen displaying a visual stimulus environment, the visual stimulus environment including switching control elements and control elements, the control elements being at least two, the visual stimulus module being provided with the possibility of creating visual stimuli, the visual stimuli being short-term modifications of the control elements and of the switching control elements displayed on the screen, the short term modifications of the control elements being at specific frequencies, each control element being associated with a corresponding target command,
the neurocomputer system being ***characterized in that:***
- the control elements are divided into at least two groups, each of said groups consists of at least one control element and has a corresponding switching control element of said switching control elements, said corresponding switching control element being associated with a command, the command being to simultaneously initiate the creation of visual stimuli for all control elements in the group to which the corresponding switching control element belongs to, and to disable the creation of visual stimuli for all control elements contained in the other groups of said at least two groups, except the creation of visual stimuli for the switching control elements of each of said other groups; and,
- the creation of visual stimuli for the control elements is organized in the course of selecting commands in the form of repeating cycles involving consecutive creation of the same visual stimuli for each of the control elements, and for selecting one target command, at least, one cycle of stimulation is used, while for selecting the command associated with the switching control element of each of the groups, a continuous creation of visual stimuli with frequencies that differ from the specific frequencies of visual stimuli of the control elements is implemented, and additionally the system detects whether there happens a fixation of the user's look in a zone of visual stimulus wherein a given switching control element is located.

Advantageously a further technical result may also be achieved by providing the identification of user's commands given to the technical device by recording electrical brain activity with an option of quick switching between two or more groups of control elements due to the following:
- creation of visual stimuli for all control elements (except switching one) during selection of commands is organized in the form of repeated cycles, including sequential creation of the same visual stimuli for each control element (except switching one) and, at least, one stimulation cycle is used to select one command; visual stimuli with frequencies that differ from frequencies of visual stimuli of all other control elements are created for selection of commands associated with switching control elements of each group, and additionally the user's gaze is fixed in visual stimulus environment, in which the selected switching control element is located;
- creation of visual stimuli for all control elements (except switching one) during selection of commands is organized in the form of repeated cycles, including sequential creation of same visual stimuli for each control element (except switching one), and at least one stimulation cycle is used for selection of one command; visual stimuli are created at frequencies other than that of visual stimuli of all other control elements after completing each stimulation cycle for control elements of each group, and additionally the user's gaze is fixed in visual stimulus environment, in which the selected switching control element is located.

Advantageously a further technical result may also be achieved due to the possibility to quickly select functional commands that can be executed for any group of control elements, because visual stimulus module contains a group of functional control elements associated with commands that can be executed for any group of control elements, which are present in visual stimulus environment. Visual stimuli for functional control elements are created in the same way as for switching control elements.

Advantageously a further technical result may also be achieved due to the fact that switching control elements are located in optimal way from the user's point of view (including in relation to other control elements), including the fact that:
- switching control elements for each group of control elements are located directly within groups to which they belong.
- switching control elements for each group of control elements are located outside groups, to which they belong, and they are grouped into at least one selected area of visual stimulus environment;
- at the time when sequential creation of visual stimuli for all control elements in specific group is initiated and performed within repeated cycles, visual stimuli are not created for switching element for this group;
- visual stimulus for switching control element is created by changing its brightness, color or content at a frequency other than that of visual stimuli of all other control elements;
- switching control elements partially or completely replace control elements contained in groups to which they belong.

The proposed invention is illustrated with the following drawings:
Figure 1 shows the scheme of the neurocomputer system for selecting commands based on brain activity recording;
Figure 2 shows a possible example of the scheme for implementing the neurocomputer system for selecting commands based on brain activity recording (illustrated by the system for typing texts).

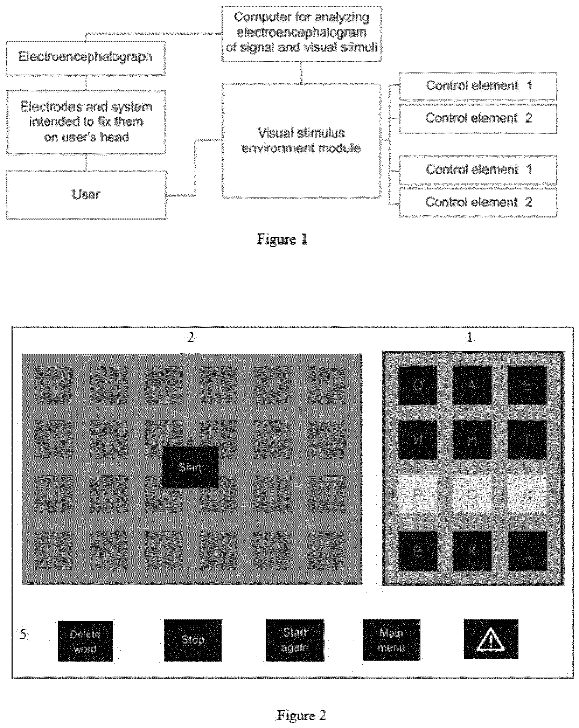

The scheme of the neurocomputer system for selecting commands based on brain activity recording shown in Figure 1 includes electroencephalograph, electrodes configured to transfer recorded pulses (including electrodes equipped with the system intended to fix them on the user's head), computer designed to analyze the recorded brain activity (electroencephalogram) in relation to the time of creation and the nature of visual stimuli, as well as visual stimulus module that combines a set of control elements (at least two control elements). Control elements are able to create visual stimuli, and each of them is associated with one command. The visual stimulus environment can be presented in various options: as a set of symbols-control elements on computer screen, as a set of moving mechanical units or as a set of LEDs.

Figure 2 shows a possible example of the scheme for implementing the neurocomputer system for selecting commands based on brain activity recording (by the example of the system for typing texts). The control elements shown in Figure 2 are divided in two groups (1 and 2). Group 1 consists of 12 control elements and group 2 includes 24 control elements. The switching between group 1 and group 2 is performed by means of command coming from switching control element. It is assumed that only one group is "active" at a certain moment of time and other matrices are "inactive", i.e. the selection of control elements in these groups is impossible at this moment.

Group 1 is "active", i.e. visual stimuli are created for all control elements of the group, except switching control element (which is hidden from the observer), just for this group at the moment shown in Figure 2. Visual stimuli during the selection of commands are created in the form of repeated cycles, including sequential creation of the same visual stimuli for each control element. The creation of visual stimuli in this example is supposed as performing short-term modifications of control elements displayed on the computer screen, for example, highlighting, increasing brightness, changing colors, replacing contents of the symbol-command, changing sizes and shape, movements, etc.

At least one stimulation cycle of control elements is used to select one command. To select a certain command, the user must focus his or her attention on the control element that is associated with the command of his or her interest (in this example, it is directly specified on the control element). Every time this control element is stimulated, the user must mentally respond to this event, due to which a specific P300 wave pattern is created in the user's electroencephalogram (fixed by the electroencephalograph and analyzed by the computer). The resulting pattern is recognized by the computer in relation to the time of creation and the nature of visual stimuli created for control elements, which allows identifying the control element to which the user is mentally responding and determine the target command in which the user is interested.

Each group of control elements in visual stimulus environment has one switching control element (for example, No. 4 in Figure 2) associated with the command that simultaneously initiates the creation of visual stimuli for all control elements in the group to which it belongs (switching element 4 refers to group 2 in Figure 2), and disables the creation of visual stimuli for all control elements contained in other groups (for example, group 1 in Figure 2), except for switching control elements of each group. At the moment the sequential creation of visual stimuli for all control elements in specific group is initiated and performed within repeated cycles (in group 1 as shown in Figure 2), visual stimuli cannot be created in relation to switching control element for this group, and moreover, this control element can be hidden from external observer (as shown in Figure 2).

It should be noted that although switching elements can be stimulated in the order of general sequence of repeated visual stimuli creation cycles, in practice such stimulation is unpractical, since it requires considerable time and does not allow achieving the goal of the proposed invention. To increase the speed and ergonomics of the neurocomputer system for selecting commands, switching control elements should be taken out from the stimulating environments of P300 matrices and control them through SSVEP-IMK system.

SSVEP-IMK is controlled by focusing the gaze on one control element that is visually stimulated (for example, changing brightness or color - "blinking") on the screen as in IMK-P300 systems. However, unlike IMK-P300, all control elements in these systems are visually stimulated not in sequential order (by turn), but simultaneously and at different frequencies (from 4 to 20 Hz on average). When focusing the gaze on control element stimulated with specific frequency, oscillations of corresponding frequency appear in the user's electroencephalogram. The computer analyzes the recorded brain activity (according to the electroencephalogram) in relation to the time of creation and the nature of visual stimuli created, and selects the command associated with control element based on matching frequencies of control element and frequencies of specific electroencephalogram peaks.

For a small number of commands, SSVEP-IMK (SSVEP-IMK) can provide a shorter command selection time than selection with the P300 Brain-Computer Interface (HMK-P300). (see Shishkin S.L., Fedorova, A.A. Nuzhdin J.O., Ganin I.P. Osadchiy A.E., Velichkovskiy B.B., Kaplan A.Y., Velichkovskiy B.M. On the way to high-speed Eye-Brain-Computer interfaces: Combination of the "One-Dimensional" paradigm and the shift of one's gaze // Bulletin of the Moscow University. Series 14: Psychology. 2013. No.4). For more than 4-6 control elements, the reliability of SSVEP-IMK (SSVEP-IMK) usage is reduced; therefore, in particular, for the example of a neuro-control system with the need for text composition, the use of a system entirely based on SSVEP-IMK would have a negative impact on the speed and accuracy of the system as a whole.

Thus, for the selection using the commands of the SSVEP-IMK system, associated with the switching control elements of each group, the continuous creation of visual stimuli with frequencies can be performed, that in its turn, is different from the visual stimulus frequencies of all other control elements, additionally accompanied by the fixation of the user's gaze in the visual zone of the stimulus environment in which the selected switching control element is located. In order to reduce the number of errors and improve the reliability of the system operation, the selection using the commands of the SSVEP-IMK system, associated with the switching control elements of each group, can also be performed not continuously but exclusively in the intervals between the P300 stimulation cycles of the control elements, after each stimulation cycle for the control elements of each group.

The grouping of the switching control elements in the visual stimulus environment depends on its content, the number of groups of control elements, and the complexity of the operations to be performed by the user. Fig. 2 illustrates a case where the switching control element 4 for the group of 2 controls is located directly within the group to which it belongs. Fig. 2 shows that the circuit of the switching control element 4 partially hides the control elements of the group 2 from the observer's view; in general, it is possible that the switching control element is completely replaced by the control elements contained within the group to which they belong. Without any restrictions, the switching control elements for each group of controls may be located outside the groups to which they belong, and they can be grouped into one or more selected areas of the visual stimulus environment.

Switching elements can also be of different sizes (it can take the place of one or more characters or cover the entire group of control elements). However, switching elements, can both replace the contents of the control elements in the group, and overlay them as a mask (without essentially affecting its content). At certain points, the switching control element (continuously or discretely) flashes in SSVEP mode - then, shifting the gaze at it, the user can select another group of control elements ("matrix"). If the system detects in the user's EEG a pattern corresponding to such a control element and its associated command, a "switching" to the matrix within which it is located occurs. This means that the current matrix, in which the stimulation of the P300 occurs, and from which the commands were selected, is "deactivated" (stimulation is ceased), and the selected new matrix is, on the contrary, "activated" (stimulation starts and it becomes available for the commands selection, from its set).

The model of the visual stimulus environment on Fig. 2 also contains a group of functional control elements associated with commands that can be executed for any group of control elements that are available in the visual stimulus environment (in the case shown on fig. 2 - groups 1 and 2), when the system works as a whole. This transfer of a set of functional commands beyond the groups of homogeneous control elements makes it possible to increase the efficiency of the system, including the implementation of the SSVEP-IMK to select data of the control elements. Functional commands can be placed in a separate area, for example, on the side or from the bottom of the main aggregate groups of control elements (illustration on fig. 2-groups 1 and 2). The efficiency of the allocation of functional commands in a separate unit depends on the availability of the commands, the need for which is relatively infrequent. A critical characteristic here is the possibility of ensuring the continuous availability of the control element associated with this command, regardless of the current state of the entire system (presence or absence of stimulation in a particular group of P300 control elements, etc.). For this, visual stimuli for functional control elements are created in the same way as for the switching control elements.

For example, Fig. 2 illustrates the case of the commands for text composition where the next elements can be set into a separate unit of the SSVEP commands: function buttons 5 related to stop and start of the stimulation in groups of P300, deleting entered characters and words, restarting the stimulation cycle in the P300, for example, if the user missed the moment of the stimulation start and wants to start over the process of the command selection. The implementation of the certain elements of the visual stimulus module is aimed at increasing the overall communication speed through the proposed system.

The following is a detailed example of the invention's implementation.

Figure 2 shows "active" 1 and "inactive" 2 matrices containing letters and signs formed on the basis of the frequency of occurrence of a particular letter or sign in Russian speech; the moment of the stimulus in the "active" matrix, when the group of control elements 3 is highlighted; switching control element 4; extra- matrix functional control elements that serve to select high-level instructions 5.

The user is placed in front of the screen, on which the control elements are displayed: command symbols grouped into at least two matrix groups. Each matrix consists of at least one control element, when in a larger number, they are grouped randomly (e.g., in the form of a regular matrix). The positions of the control elements inside the matrix are fixed. To select commands from a matrix, the matrix needs to be "active", i.e., stimuli must be presented in it (at each control stage only one matrix of those present in the user's field of view is "active").

Stimuli are short-term modifications of control elements, i.e. backlighting (brightness increase), color change, replacing the contents of the command symbol, changing the size and shape, movement, etc. Stimulation is carried out in such a way that at every instant the changes occur with one element or with a group of matrix elements; Thus, stimulation is the successive modifications of individual elements or groups of elements of a matrix. Each stage of work with the system corresponds to the selection of one command from the elements of the matrix. Within this stage, each of the controls is subjected to stimulation at least once. However, in most cases, to select a single command, stimulation of the control element must be repeated several times. The user is shown in advance how the stimulation is effected.

To select a command from the matrix, the user must focus his/her attention on the position where the desired (target) element of the "active" matrix is located, wait for the stimulation to begin, and then mentally respond to the moment the stimulus appears in the target position, ignoring the stimuli in the other positions of the matrix, if there are any. The type of mental response to the stimulus in the target position is indicated to the user in advance, the most common instruction option reads as follows: "Mentally, clearly read the appearance of the target stimulus" (note: of the stimulus in the target position of the matrix). The target stimulus can be indicated to the user in advance (for example, by the researcher), or the user can arbitrarily decide for himself/herself which stimulus is the target.

In the process of completing this task, the activity of the user's brain is registered non-invasively. In the most common case, activity is recorded using the electroencephalography (EEG) method in a standard procedure. The system processes the brain signals, and after completion of each stage of work with stimuli (end of stimulation) produces a result. The result is a recognized control element coupled with the associated command. The result can be presented to the end user in various forms. For example, when a letter or another graphic symbol is selected, the recognized symbol is highlighted, an additional action may occur (including, when typing a text, the selected letter being typed to a special result row). Also, the system operation can result in the non-recognition of a command (e.g., if the user for some reason failed to focus or poorly focused on the target stimulus); in this case, none of the control elements is recognized by the system.

The elements of the "inactive" matrix (matrices) are not stimulated at the stage of the user's work with the "active" matrix. The user quickly switches to another "inactive" matrix using the switching controls. When the user selects the appropriate switching control element, the "inactive" matrix with which this switching element is associated becomes "active", and the matrix with which the operation was performed, on the contrary, changes from the "active" state to the "inactive" state.

For a switching control element to be selected, it must be in the "active" state at the time of the selection, which involves changing the element's state ("flashing") at a specified frequency. The "flashing" frequency is adjusted for the system in a certain way, in the most typical case lies in the range of 4-20 Hz. To select the switching control, the user must shift his/her look at this element when it is "active" ("flashing") and focus it on this element for some time. The user is told in advance the duration of this time, which is normally 4-10 seconds. It is worth noting that the switching control element can be either simple (e.g., take the place of only one element from the "inactive" matrix) or complex (consisting of more than one element of the matrix, or be the size of an entire matrix). In addition, the switching control element, in particular, can both replace the contents of the elements of the "inactive" matrix and keep all or part of the elements unchanged.

In one of the system implementation variants, this control element can be one or more of the original cells of the "inactive" matrix in which it is located, and its flashing can represent the same modifications of the "inactive" matrix elements that occur in it during regular stimulation, when it is used for control in its "active" state (e.g., when backlit), but at a preset constant frequency. In another variant, the switching control element can be a special symbol that is located in one or more positions of the "inactive" matrix, replacing with itself the contents of the corresponding matrix elements.

When an associated matrix is activated by the corresponding switching control element, this matrix becomes "active", therefore it becomes possible to select a command from among the number of control elements contained in this matrix. In addition, the switching control element that was used to do the switching can either subsequently be displayed within the given matrix or not be displayed at all. The former "active" matrix is simultaneously "deactivated", i.e. its elements can no longer be selected until this matrix is "activated" again. For activation, a switching control object with the properties described above appears in this "inactive" matrix. If more than two matrices are present on the screen (i.e., there are more than one "inactive" matrices at any given time), but such switching control elements are present in all "inactive" matrices.

In addition to the switching control elements within the "inactive" matrices, similar functional control elements can be located on the screen outside one of the matrices. Their purpose can be to quickly select some of the most important and at the same time infrequently used commands. For example, when printing text, these functional control elements may be responsible for deleting the last letter or word entered, "restarting" the stimulation in the "active matrix" (i.e. initiating the beginning of a new command selection stage). In systems for patients with motor impairments, such functional controls can respond to vital commands or perform the function of an "alarm button". Similarly, to the switching control elements, these individual functional elements can be "active" (i.e., provide a command selection using them) both during the whole system operation time and can be active only at certain stages (for example, in the time gaps between the stages of selecting control elements from matrices).

The advantage of the proposed neural computer system for selecting commands based on the registration of brain activity is the ability to split a set of all controls into several groups (matrices) simultaneously present on the screen and to provide the possibility of a quick switching between them using switching controls. The proposed approach to the organization of a visual stimulus environment allows selecting a team from individual matrices more quickly than from a single matrix that would contain as many control elements as the total is contained in individual matrices. In this case, switching between matrices is a command whose speed of selection is provided by algorithms different from those used in multicomputer matrices.

In addition, non-matrix functional controls working on the basis of the same algorithms can provide a quick selection of other important commands that are homogeneous for groups of controls within matrices. For example, as noted above, it is possible to select control commands in the course of printing of the text. In addition, using the functional controls, one can select the navigation commands for the entire system: move between the different screens (containing different matrices with control elements, go to the basic or main sections of the interface control menu, where one can also select different control matrices).

This system of switching between different groups of controls can be used not only for "mental" typing, but also, for example, as a virtual TV control panel, or "smart house". When controlling the TV in the same way as when entering texts, it can be necessary to select frequently used commands (for example, frequent TV channels) in a separate matrix with the ability to quickly select these commands, as well as the ability to switch to the other matrices (rare TV channels, settings) or moving to other sections of the TV control menu. The last two switching options can be implemented using "fast" switching or functional controls, while the selection of the basic commands (navigation via TV channels) can be carried out using elements in the main control matrices. When using such a control system in the "smart house", the user can select various commands (switching on lighting, controlling the TV, the music center, turning on the computer or activating the phone) with the help of basic controls, and at the same time, using switching and functional control elements quickly switch to other multi-command control matrices. For example, to print text when using a computer, to enter a number when using the phone, or to select TV channels when turning on the TV. In turn, the menu of each of the devices in these examples can have its own sections, controlled by basic commands in the matrices, and navigation between sections can be carried out using the "fast" switching and functional controls (for example, controlling the TV).

The practical application of the present invention has been discussed above; the useful effects of applying the present invention consist in general acceleration of the user's process of selecting commands (decreasing the time for selecting one command and reducing the probability of an error when selecting a command from a matrix with fewer elements). In control systems which require a large number of control elements (several dozens), for example, for "mental" typing, it is advisable to organize a matrix of small sizes with the most frequently used commands (private letters), as well as the other matrices (with rare letters, numbers, punctuation marks, another language layout, etc.).

Switching between such matrices is potentially possible using the same algorithms which are used to select the control elements in them. However, first, it is longer due to the need to select a switching command out of a variety of the other commands. Secondly, it is less ergonomic for the user, since the switching element is outside the object, for the "activation" of which it is responsible.

The proposed system allows overcoming these shortcomings, providing the ability to quickly switch between groups of controls, in some implementations possible at any time (even at the stage of selecting a command from the matrix). In addition, the switching process itself is organized more logically, because to activate the required set of commands (matrix), the user only needs to transfer and fix a look at the element (s) of this matrix. In some implementations of the present system, the functional controls located separately (outside the matrices) can also speed up the user's work in the system, and also enable him to quickly select some important commands

The scope of the invention is defined by the appended claims.

## Claims

1. A neurocomputer system for selecting commands based on recording brain activity, comprising:
- an electroencephalograph;
- electrodes configured to transmit detected pulses;
- an electronic computing device for analyzing the recorded brain activity; and
- a visual stimulus module that includes a screen displaying a visual stimulus environment, the visual stimulus environment including switching control elements and control elements, the control elements being at least two, the visual stimulus module being provided with the possibility of creating visual stimuli, the visual stimuli being short-term modifications of the control elements and of the switching control elements displayed on the screen, the short term modifications of the control elements being at specific frequencies, each control element being associated with a corresponding target command,
the neurocomputer system being ***characterized in that:***
- the control elements are divided into at least two groups, each of said groups consists of at least one control element and has a corresponding switching control element of said switching control elements, said corresponding switching control element being associated with a command, the command being to simultaneously initiate the creation of visual stimuli for all control elements in the group to which the corresponding switching control element belongs to, and to disable the creation of visual stimuli for all control elements contained in the other groups of said at least two groups, except the creation of visual stimuli for the switching control elements of each of said other groups; and,
- the creation of visual stimuli for the control elements is organized in the course of selecting commands in the form of repeating cycles involving consecutive creation of the same visual stimuli for each of the control elements, and for selecting one target command, at least, one cycle of stimulation is used, while for selecting the command associated with the switching control element of each of the groups, a continuous creation of visual stimuli with frequencies that differ from the specific frequencies of visual stimuli of the control elements is implemented, and additionally the system detects whether there happens a fixation of the user's look in a zone of visual stimulus wherein a given switching control element is located.

2. A neurocomputer system according to claim 1, **characterized in that** the visual stimulus environment comprises a group of functional control elements associated with functional commands the execution of which is possible for any group of control elements present in the visual stimulus environment, while the creation of visual stimuli for the functional control elements is carried out in the same way as it is performed for the switching control elements.

3. Neurocomputer system according to claim 1, wherein in the visual stimulus environment the switching control elements for each group of control elements are located directly within the groups to which they belong.

4. Neurocomputer system according to claim 1, **characterized in that** the switching control elements for each group of control elements are outside the groups to which they belong and are grouped in at least one selected area of the visual stimulus environment.

5. Neurocomputer system according to claim 1, wherein at the moment, when the consecutive creation of visual stimuli for all control elements in a certain group is initiated and implemented in the framework of repeated cycles, visual stimuli are not created with respect to the switching control element for said certain group.

6. Neurocomputer system according to claim 1, wherein a visual stimulus for the switching control element is created by changing the latter's brightness, color or content with a frequency other than that of the visual stimuli of all other control elements.

7. Neurocomputer system according to claim 5, wherein in the displayed visual stimulus environment the switching control elements displayed on the screen partially hide from an observer's view, or overlay as a mask and replace from the observer's view, the control elements contained in the groups to which they relate.

## Patentansprüche

1. Neurocomputersystem zum Auswählen von Befehlen basierend auf der Aufzeichnung der Gehirnaktivität, umfassend:
- einen Elektroenzephalographen;
- Elektroden, die konfiguriert sind, um erkannte Impulse zu übertragen;
- eine elektronische Rechenvorrichtung zum Analysieren der aufgezeichneten Gehirnaktivität; und
- ein Modul für visuelle Reize, das einen Bildschirm einschließt, der eine Umgebung für visuelle Reize anzeigt, wobei die Umgebung für visuelle Reize Schaltsteuerelemente und Steuerelemente einschließt, wobei die Steuerelemente mindestens zwei sind, wobei das Modul für visuelle Reize mit der Möglichkeit versehen ist, visuelle Reize zu erzeugen, wobei die visuellen Reize kurzzeitige Modifikationen der Steuerelemente und der auf dem Bildschirm angezeigten Schaltsteuerelemente sind, wobei die kurzzeitigen Modifikationen der Steuerelemente bei bestimmten Frequenzen stattfinden, wobei jedem Steuerelement ein entsprechender Zielbefehl zugeordnet ist,
wobei das Neurocomputersystem ***dadurch gekennzeichnet ist, dass:***
- die Steuerelemente in mindestens zwei Gruppen unterteilt sind, wobei jede der Gruppen aus mindestens einem Steuerelement besteht und ein entsprechendes Schaltsteuerelement der Schaltsteuerelemente aufweist, wobei das entsprechende Schaltsteuerelement einem Befehl zugeordnet ist, wobei der Befehl darin besteht, gleichzeitig die Erzeugung von visuellen Reizen für alle Steuerelemente in der Gruppe zu initiieren, zu der das entsprechende Schaltsteuerelement gehört, und die Erzeugung von visuellen Reizen für alle Steuerelemente zu deaktivieren, die in den anderen Gruppen der mindestens zwei Gruppen enthalten sind, mit Ausnahme der Erzeugung visueller Reize für die Schaltsteuerelemente jeder der anderen Gruppen; und,
- die Erzeugung von visuellen Reizen für die Steuerelemente im Zuge der Auswahl von Befehlen in Form von sich wiederholenden Zyklen organisiert ist, bei denen aufeinanderfolgend die gleichen visuellen Reize für jedes der Steuerelemente erzeugt werden, und zum Auswählen eines Zielbefehls mindestens eines Stimulationszyklus verwendet wird, während zum Auswählen des Befehls, der dem Schaltsteuerelement jeder der Gruppen zugeordnet ist, eine kontinuierliche Erzeugung visueller Reize mit Frequenzen umgesetzt wird, die sich von den spezifischen Frequenzen visueller Reize der Steuerelemente unterscheiden, und außerdem das System erkennt, ob ein Fixierung des Blicks des Benutzers in einer Zone des visuellen Reizes stattfindet, in der sich ein gegebenes Schaltsteuerelement befindet.

2. Neurocomputersystem nach Anspruch 1, **dadurch gekennzeichnet, dass** die Umgebung für visuelle Reize eine Gruppe von funktionellen Steuerelementen umfasst, die funktionellen Befehlen zugeordnet sind, deren Ausführung für jede Gruppe von Steuerelementen möglich ist, die in der Umgebung für visuelle Reize vorhanden sind, während die Erzeugung von visuellen Reizen für die funktionellen Steuerelemente in gleicher Weise wie für die Schaltsteuerelemente durchgeführt wird.

3. Neurocomputersystem nach Anspruch 1, wobei sich in der Umgebung für visuelle Reize die Schaltsteuerelemente für jede Gruppe von Steuerelementen direkt innerhalb der Gruppen befinden, zu denen sie gehören.

4. Neurocomputersystem nach Anspruch 1, **dadurch gekennzeichnet, dass** die Schaltsteuerelemente für jede Gruppe von Steuerelementen außerhalb der Gruppen liegen, zu denen sie gehören, und in mindestens einem ausgewählten Bereich der Umgebung für visuelle Reize gruppiert sind.

5. Neurocomputersystem nach Anspruch 1, wobei in dem Moment, in dem die fortlaufende Erzeugung von visuellen Reizen für alle Steuerelemente einer bestimmten Gruppe initiiert und im Rahmen von wiederholten Zyklen durchgeführt wird, keine visuellen Reize in Bezug auf das Schaltsteuerelement für diese bestimmte Gruppe erzeugt werden.

6. Neurocomputersystem nach Anspruch 1, wobei ein visueller Reiz für das Schaltsteuerelement erzeugt wird, indem dessen Helligkeit, Farbe oder Inhalt mit einer anderen Frequenz als derjenigen der visuellen Reize aller anderen Steuerelemente verändert wird.

7. Neurocomputersystem nach Anspruch 5, wobei in der angezeigten Umgebung für visuelle Reize die auf dem Bildschirm angezeigten Schaltsteuerelemente teilweise aus der Sicht eines Betrachters ausgeblendet oder als Maske überlagert werden und aus der Sicht des Betrachters die in den Gruppen enthaltenen Steuerelemente ersetzen, auf die sie sich beziehen.

## Revendications

1. Système informatique neuronal pour choisir des instructions sur la base de l'enregistrement de l'activité cérébrale, comprenant :
- un électroencéphalographe ;
- des électrodes configurées pour transmettre des impulsions détectées ;
- un dispositif informatique électronique pour analyser l'activité cérébrale enregistrée ; et
- un module de stimulation visuelle qui comporte un écran affichant un milieu de stimulation visuelle, le milieu de stimulation visuelle comportant des éléments de commutation de commande et des éléments de commande, les éléments de commande étant au moins deux, le module de stimulation visuelle étant pourvu de la possibilité de créer des stimuli visuels, les stimuli visuels étant des modifications à court terme des éléments de commande et des éléments de commutation de commande affichés sur l'écran, les modifications à court terme des éléments de commande étant à des fréquences spécifiques, chaque élément de commande étant associé à une instruction cible correspondante,
le système informatique neuronal étant ***caractérisé en ce que :***
- les éléments de commande sont répartis en au moins deux groupes, chacun desdits groupes est constitué d'au moins un élément de commande et possède un élément de commutation de commande correspondant parmi lesdits éléments de commutation de commande, ledit élément de commutation de commande correspondant étant associé à une instruction, l'instruction étant d'initier simultanément la création de stimuli visuels pour tous les éléments de commande du groupe auquel appartient l'élément de commutation de commande correspondant, et de désactiver la création de stimuli visuels pour tous les éléments de commande contenus dans les autres groupes desdits au moins deux groupes, à l'exception de la création de stimuli visuels pour les éléments de commutation de commande de chacun desdits autres groupes ; et,
- la création de stimuli visuels pour les éléments de commande est organisée au cours de la sélection d'instructions sous forme de cycles répétitifs impliquant la création consécutive des mêmes stimuli visuels pour chacun des éléments de commande, et pour la sélection d'une instruction cible, au moins, un cycle de stimulation est utilisé, tandis que pour sélectionner l'instruction associée à l'élément de commutation de commande de chacun des groupes, une création continue de stimuli visuels avec des fréquences qui diffèrent des fréquences spécifiques de stimuli visuels des éléments de commande est mise en œuvre, et en outre le système détecte s'il se produit une fixation du regard de l'utilisateur dans une zone de stimulation visuelle dans laquelle un élément de commutation de commande donné est situé.

2. Système informatique neuronal selon la revendication 1, **caractérisé en ce que** le milieu de stimulation visuelle comprend un groupe d'éléments de commande fonctionnels associés à des instructions fonctionnelles dont l'exécution est possible pour tout groupe d'éléments de commande présent dans le milieu de stimulation visuelle, tandis que la création de stimuli visuels pour les éléments de commande fonctionnels est effectuée de la même manière que pour les éléments de commutation de commande.

3. Système informatique neuronal selon la revendication 1, dans lequel dans le milieu de stimulation visuelle, les éléments de commutation de commande pour chaque groupe d'éléments de commande sont situés directement dans les groupes auxquels ils appartiennent.

4. Système informatique neuronal selon la revendication 1, **caractérisé en ce que** les éléments de commutation de commande pour chaque groupe d'éléments de commande sont en dehors des groupes auxquels ils appartiennent et sont regroupés dans au moins une zone sélectionnée du milieu de stimulation visuelle.

5. Système informatique neuronal selon la revendication 1, dans lequel au moment où la création consécutive de stimuli visuels pour tous les éléments de commande d'un certain groupe est initiée et mise en œuvre dans le cadre de cycles répétés, les stimuli visuels ne sont pas créés par rapport à l'élément de commutation de commande pour ledit certain groupe.

6. Système informatique neuronal selon la revendication 1, dans lequel un stimulus visuel pour l'élément de commutation de commande est créé en changeant la luminosité, la couleur ou le contenu de ce dernier avec une fréquence autre que celle des stimuli visuels de tous les autres éléments de commande.

7. Système informatique neuronal selon la revendication 5, dans lequel dans le milieu de stimulation visuelle affiché, les éléments de commutation de commande affichés sur l'écran cachent partiellement de la vue d'un observateur, ou se superposent comme un masque et remplacent de la vue de l'observateur, les éléments de commande contenus dans les groupes auxquels ils se rapportent.
